(19) Europäisches Patentamt
European Patent Office
Office européen des brevets

(11) Veröffentlichungsnummer: **0 126 296**
**B1**

# (12) EUROPÄISCHE PATENTSCHRIFT

(45) Veröffentlichungstag der Patentschrift: **01.06.88**

(51) Int. Cl.⁴: **C 07 D 239/36**, A 61 K 31/505

(21) Anmeldenummer: **84104324.3**

(22) Anmeldetag: **17.04.84**

(54) **Substituierte Phenyl-2-(1H)-pyrimidinone, Verfahren zu ihrer Herstellung und ihre Verwendung sowie diese Verbindungen enthaltende Zubereitungen.**

(30) Priorität: **30.04.83 DE 3315797**

(43) Veröffentlichungstag der Anmeldung: **28.11.84 Patentblatt 84/48**

(45) Bekanntmachung des Hinweises auf die Patenterteilung: **01.06.88 Patentblatt 88/22**

(84) Benannte Vertragsstaaten: **AT BE CH DE FR GB IT LI NL SE**

(56) Entgegenhaltungen:
**CHIMIE THERAPEUTIQUE, Band 5, Nr. 4, Juli-August 1970, Seiten 264-269, Paris, FR; K. TAKAGI et al.: "Recherche d'agents anti-tumoraux. IV. Sur la formation de nouvelles pyrimidines à partir de benzofurannes substitués en position 3 par un groupement électro-attractif"**
**CHIMIE THERAPEUTIQUE, Nr. 3, Mai-Juni 1973, Seiten 314-318, Paris, FR; M. HUBERT-HABART et al.: "Recherches sur les dérivés nitrés d'intérêt biologique. VI. - Sur la synthèse d'(hydroxy-2 nitro-4 phényl)-5 pyrimidines à partir de dérivés nitrés de benzofurannes subsitués en 3 par un groupement électro-attractif"**

(73) Patentinhaber: **Beiersdorf Aktiengesellschaft, Unnastrasse 48, D-2000 Hamburg 20 (DE)**

(72) Erfinder: **Stenzel, Wolfgang, Dr., Lerchenweg 87, D-2057 Reinbek (DE)**

**EP 0 126 296 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Substituierte Phenyl-2-(1H)-pyrimidinone, Verfahren zu ihrer Herstellung und ihre Verwendung sowie diese Verbindungen enthaltende Zubereitungen

Gegenstand der Erfindung sind substituierte Phenyl-2-(1H)-pyrimidinone der allgemeinen Formel I

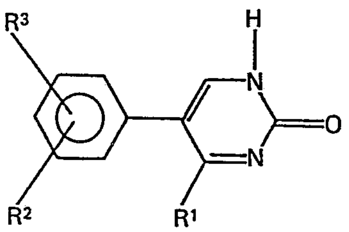

(I)

in der

$R^1$ eine Alkylgruppe mit 1 bis 4 Kohlenstoffatomen und $R^2$ und $R^3$, die gleich oder verschieden sein können, jeweils Wasserstoff, Halogen, Cyano, eine Trifluormethylgruppe, eine Hydroxylgruppe oder eine Alkoxygruppe mit 1 bis 4 Kohlenstoffatomen bedeuten, wobei Alkylteile jeweils geradkettig oder verzweigt sein können sowie ihre tautomeren Formen und ihre Säureadditionssalze und N-Oxide, Verfahren zu ihrer Herstellung und ihre Verwendung in pharmazeutischen Präparaten.

Der Einfachheit halber sind die erfindungsgemässen Verbindungen in nur einer durch die allgemeine Formel I wiedergegebenen tautomeren Form definiert. Die Erfindung erstreckt sich jedoch auf alle tautomeren Formen der Verbindungen.

Die Verbindung 5-(2-Hydroxyphenyl)-4-ethyl-2-(1H)-pyrimidinon wurde bereits in der Literatur (Chimie Therapeutique 5 (1970), 267) ohne Wirkungsangabe genannt.

Obgleich pharmazeutisch verträgliche Salze der Verbindungen der allgemeinen Formel I und deren tautomere Formen und N-Oxide bevorzugt sind, liegen alle Säureadditionssalze innerhalb des Bereichs der Erfindung. Alle Säureadditionssalze sind wertvoll zur Herstellung der Basen, selbst wenn das spezielle Salz nur als Zwischenprodukt gewünscht wird, wie z.B., wenn das Salz nur für Zwecke der Reinigung oder Identifizierung gebildet wird, oder wenn es als ein Zwischenprodukt bei der Herstellung eines pharmazeutisch verträglichen Salzes, z.B. durch Ionenaustauschverfahrensweisen, verwendet wird.

Die Erfindung bezieht sich auch auf die N-Oxide der Verbindungen der allgemeinen Formel I. Sie sind nach bekannten Herstellungsverfahren erhältlich (H.S. Mosher et al., Org. Synth., Coll. Vol. IV, 828, 1963).

Die Alkylgruppen sowie die Alkylteile der Alkoxygruppen können geradkettig oder verzweigt sein.

Besonders bevorzugte Alkylgruppen $R^1$ sind die Methyl- und Ethylgruppe.

Die Reste $R^2$ und $R^3$ befinden sich vorzugsweise in der 3-Stellung und/oder 4-Stellung des Phenylrests.

Halogen ist vorzugsweise Fluor oder Chlor.

Bevorzugte Alkoxygruppen sind Methoxy- und Ethoxygruppen.

Die folgenden Verbindungen der allgemeinen Formel I, deren N-Oxide und deren Salze werden besonders bevorzugt:

5-(3-Hydroxyphenyl)-4-methyl-2-(1H)-pyrimidinon-hydrobromid
5-(3-Hydroxyphenyl)-4-ethyl-2-(1H)-pyrimidinon-hydrobromid
5-(3-Methoxyphenyl)-4-methyl-2-(1H)-pyrimidinon
5-(3-Chlorphenyl)-4-methyl-2-(1H)-pyrimidinon
5-(4-Hydroxyphenyl)-4-methyl-2-(1H)-pyrimidinon-hydrobromid
5-(4-Hydroxyphenyl)-4-ethyl-2-(1H)-pyrimidinon-hydrobromid
5-(4-Methoxyphenyl)-4-methyl-2-(1H)-pyrimidinon
5-(4-Chlorphenyl)-4-methyl-2-(1H)-pyrimidinon.

Die erfindungsgemässen Substanzen, ihre physiologisch verträglichen Säureadditionssalze und N-Oxide besitzen wertvolle pharmakologische Eigenschaften, insbesondere eine positiv inotrope Wirkung, und sind daher zur Behandlung von Herzinsuffizienz geeignet.

Die Verbindungen der Erfindung können beim Menschen oral oder parenteral in einer Dosierung von 10–1200 mg, vorzugsweie 100–800 mg, besonders bevorzugt 300–600 mg pro Tag, angewendet werden, insbesondere in unterteilten Dosen, zum Beispiel dreimal täglich. Diese Dosierung gilt besonders für die Behandlung von Herzinsuffizienz.

Die positiv inotrope Wirkung der erfindungsgemässen Verbindungen wurde am Meerschweinchen-Papillarmuskel bestimmt [Naunyn-Schmiedeberg's Arch. Pharmacol. 304, 37 (1978)]. Die Konzentration der Substanz im Organbad betrug jeweils $3 \times 10^{-4}$ mol/l. Die maximale prozentuale Steigerung der Kontraktionsamplitude wurde jeweils an drei Papillarmuskeln bestimmt und betrug mindestens 50%.

Gemäss der Erfindung werden pharmazeutische Zusammensetzungen geschaffen, die eine Verbindung der allgemeinen Formel I und deren N-Oxide oder deren pharmazeutisch verträgliche Salze zusammen mit einem pharmazeutisch verträglichen Verdünnungsmittel oder Träger enthalten.

Die Verbindungen gemäss der Erfindung können mit üblichen pharmazeutisch verträglichen Verdünnungsmitteln oder Trägern und gegebenenfalls mit anderen Hilfsmitteln vermischt und beispielsweise oral oder parenteral verabreicht werden. Sie können oral in Form von Tabletten, Dragees, Sirups, Suspensionen und Flüssigkeiten oder parenteral in Form von Lösungen oder Suspensionen verabreicht werden. Oral zu verabreichende Präparate können einen oder mehrere Zusätze wie Süssungsmittel, Aromatisierungsmittel, Farbstoffe und Konservierungsmittel enthalten. Tabletten können den Wirkstoff mit üblichen pharmazeutisch verträglichen Hilfsmitteln vermischt enthalten, zum Beispiel inerten Verdünnungsmitteln wie Calciumcarbonat, Natriumcarbonat, Lactose und Talk, Granulierungsmitteln und Mitteln, die den Zerfall der Tabletten bei oraler Verabreichung fördern wie Stärke oder Alginsäure, Bindemitteln wie Stärke, oder Gelatine, Gleitmitteln wie Magnesiumstearat, Stearinsäure und Talk.

Geeignete Trägerstoffe sind beispielsweise Milchzucker (Lactose), Gelatine, Maisstärke, Stearinsäure, Ethanol, Propylenglycol, Ether des Tetrahydrofurylalkohols und Wasser.

Die Tabletten können nach bekannten Arbeitsweisen überzogen werden, um den Zerfall und die Resorption im Magen-Darmtrakt zu verzögern, wodurch die Aktivität des Wirkstoffs sich über eine längere Zeitspanne erstrecken kann. Ebenso kann in den Suspensionen der Wirkstoff mit Hilfsmitteln vermischt sein, die für die Herstellung solcher Zusammensetzungen üblich sind, zum Beispiel Suspendiermitteln wie Methylcellulose, Tragacanth oder Natriumalginat, Netzmitteln wie Lecithin, Polyethylenstearat und Polyoxyethylensorbitanmonooleat, und Konservierungsmitteln wie Ethylparahydroxybenzoat. Kapseln können den Wirkstoff als einzigen Bestandteil oder vermischt mit einem festen Verdünnungsmittel wie Calciumcarbonat, Calciumphosphat oder Kaolin enthalten. Die injizierbaren Präparate werden ebenfalls in an sich bekannter Weise formuliert. Die pharmazeutischen Präparate können den Wirkstoff in einer Menge von 0,1 bis 90%, insbesondere 1 bis 90%, enthalten, wobei der Rest ein Trägerstoff oder Zusatzstoff ist. Im Hinblick auf die Herstellung und Verabreichung werden feste Präparate wie Tabletten und Kapseln bevorzugt. Vorzugsweise enthalten die Präparate den Wirkstoff in einer Menge von 100 mg.

Die Verbindungen der allgemeinen Formel I können hergestellt werden durch Umsetzung von Verbindungen der allgemeinen Formel II

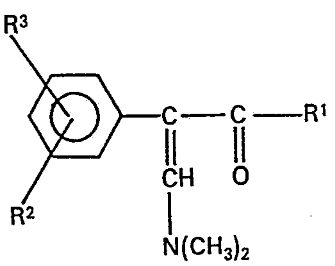

(II)

in der
$R^1$ eine Alkylgruppe mit 1 bis 4 Kohlenstoffatomen und $R^2$ und $R^3$, die gleich oder verschieden sein können, jeweils Wasserstoff, Halogen, Cyano, eine Trifluormethylgruppe, eine Hydroxylgruppe oder eine Alkoxygruppe mit 1 bis 4 Kohlenstoffatomen bedeuten, wobei Alkylteile jeweils geradkettig oder verzweigt sein können, mit Harnstoff.

Die Reaktion der Verbindungen der allgemeinen Formel II mit Harnstoff erfolgt vorzugsweise durch Erhitzen in einem geeigneten Lösungsmittel wie Methanol, Ethanol oder Dimethylformamid in Gegenwart eines basischen Kondensationsmittels, vorzugsweise eines Alkalimetallalkoholats wie Natriumethylat. Andere geeignete Lösungsmittel sind Toluol, Dioxan, Acetonitril oder Tetrahydrofuran. Die Reaktionstemperaturen liegen zwischen 65 und 150°C. Die Reaktionszeiten variieren nach Wahl der Reaktionsbedingungen zwischen 8 und 48 Stunden. Die optimalen Reaktionszeiten werden zweckmässigerweise durch Dünnschichtchromatographie an Kieselgel bestimmt.

Verbindungen der allgemeinen Formel I, in denen $R^2$ oder $R^3$ eine Alkoxygruppe bedeutet können durch Etherspaltung in die entsprechenden erfindungsgemässen Hydroxy-Derivate überführt werden. Zur Etherspaltung verwendet man vorzugsweise Bromwasserstoffsäure oder auch andere für diese Reaktion geeignete Reagenzien wie Bortribromid oder Trimethylsilyljodid.

Die Verbindungen der allgemeinen Formel II können aus Arylketonen der allgemeinen Formel III

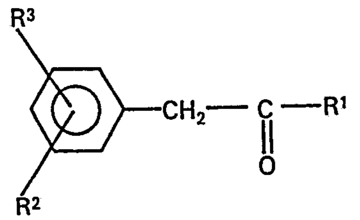

(III)

in der
$R^1$ eine Alkylgruppe mit 1 bis 4 Kohlenstoffatomen und $R^2$ und $R^3$, die gleich oder verschieden sein können, jeweils Wasserstoff, Halogen, Cyano, eine Trifluormethylgruppe, eine Hydroxylgruppe oder eine Alkoxygruppe mit 1 bis 4 Kohlenstoffatomen bedeuten, wobei Alkylteile jeweils geradkettig oder verzweigt sein können, durch Umsetzung mit Dimethylformamiddimethylacetal hergestellt werden.

Die Reaktionen können in einem Lösungsmittel wie Dimethylformamid oder auch ohne Lösungsmittel bei Temperaturen zwischen 50°C und der Siedetemperatur des Reaktionsgemisches, vorzugsweise zwischen 70 und 100°C durchgeführt werden.

Arbeitet man ohne Lösungsmittel mit einem Überschuss an Dimethylformamiddimethylacetal bei 70 bis 100°C, liegen die Reaktionszeiten zwischen einer und acht Stunden.

Die bei dem Verfahren verwendeten Ausgangsverbindungen sind bekannt oder können nach bekannten Methoden hergestellt werden.

Die Verbindungen der allgemeinen Formel I können entweder als Basen oder in der Form ihrer Salze aus den Reaktionsgemischen isoliert werden. Sie lassen sich als Basen mit geeigneten anorganischen oder organischen Säuren nach bekannten Verfahren in Salze überführen.

Bevorzugt werden physiologisch verträgliche Salze. Hierfür sind als anorganische Säuren beispielsweise Halogenwasserstoffsäuren, zum Beispiel Salzsäure, oder Schwefelsäure, und als organische Säuren zum Beispiel Fumarsäure, Maleinsäure, Citronensäure und Weinsäure geeignet. Zur Herstellung wird die heisse alkalische Lösung der Base mit der alkoholischen Lösung einer geeigneten Säure versetzt, und man erhält nach Etherzusatz das Salz.

Die folgenden Beispiele dienen zur Erläuterung der Erfindung:

Beispiel 1

5-(4-Hydroxyphenyl)-4-methyl-2-(1H)-pyrimidinon-hydrobromid

1,5 g 5-(4-Methoxyphenyl)-4-methyl-2-(1H)-pyrimidinon werden mit 12,5 ml 47-prozentiger Bromwasserstoffsäure 24 Stunden auf 100°C erhitzt. Danach wird auf 0°C abgekühlt, das auskristallisierte Produkt abgesaugt und mit kaltem Ethanol gewaschen. Man erhält 1,3 g 5-(4-Hydroxyphenyl)-4-methyl-2-(1H)-pyrimidinon-hydrobromid. Schmp. über 300°C.

Beispiel 2

5-(4-Methoxyphenyl)-4-methyl-2-(1H)-pyrimidinon

35 g 3-(4-Methoxyphenyl)-2-propanon und 70 ml Dimethylformamiddimethylacetal werden 3 Stunden auf 70°C erhitzt. Danach wird das Reaktionsgemisch im Vakuum bis zur Trockne eingeengt, der Rückstand in Petrolether zur Kristallisation gebracht und abgesaugt. Man erhält 43 g 4-Dimethylamino-3-(4-methoxyphenyl)-3-buten-2-on. Schmp. 51°C.

Eine Lösung von 40,0 g 4-Dimethylamino-3-(4-methoxyphenyl)-3-buten-2-on in 120 ml Ethanol wird mit 24,4 g Harnstoff und 120 ml einer 25-prozentigen ethanolischen Natriumethylatlösung versetzt. Man erhitzt 8 Stunden bei Rückflusstemperatur, kühlt auf 0°C, saugt das Reaktionsprodukt ab und wäscht mit kaltem Ethanol nach. Der Rückstand wird in Wasser gelöst und unter Eiskühlung mit verdünnter Salzsäure neutralisiert. Das ausgefallene Produkt wird abgesaugt, mit Wasser nachgewaschen und getrocknet. Man erhält 19,5 g 5-(4-Methoxyphenyl)-4-methyl-2-(1H)-pyrimidinon. Schmp. 210°C.

3,5 g 5-(4-Methoxyphenyl)-4-methyl-2-(1H)-pyrimidinon werden in Methanol gelöst, mit etherischer Salzsäure angesäuert (pH 2–3) und bis zur beginnenden Trübung mit Ether versetzt. Man kühlt und saugt die Kristalle ab. Nach dem Trocknen erhält man 3,3 g 5-(4-Methoxyphenyl)-4-methyl-2-(1H)-pyrimidinon-hydrochlorid. Schmp. 265°C (Zers.)

Beispiel 3

5-(2-Hydroxyphenyl)-4-methyl-2-(1H)-pyrimidinon-hydrochlorid

10,0 g 5-(2-Methoxyphenyl)-4-methyl-2-(1H)-pyrimidinon werden mit 100 ml 40-prozentiger Bromwasserstoffsäure in Eisessig versetzt und 48 Stunden unter Rückfluss erhitzt. Anschliessend wird eingedampft und der Rückstand mit Ether verrieben und abgesaugt. Das Rohprodukt wird in 100 ml Wasser gelöst, mit Aktivkohle entfärbt und mit verdünnter Natronlauge neutralisiert. Der Niederschlag wird abgesaugt, getrocknet, in Methanol gelöst, mit etherischer Salzsäure auf pH 2 eingestellt und bis zur beginnenden Trübung mit Ether versetzt. Anschliessend wird gekühlt, abgesaugt und der Rückstand aus Ethanol umkristallisiert. Man erhält 2,5 g 5-(2-Hydroxyphenyl)-4-methyl-2-(1H)-pyrimidinon-hydrochlorid. Schmp. 241–243°C (Zers.)

Herstellung von Tabletten und Kapseln

Tabletten und Kapseln, welche die nachstehend angegebenen Bestandteile enthalten, werden nach bekannten Arbeitsweisen hergestellt. Diese sind für die Behandlung von Herzinsuffizienz in Dosierungsmengen von jeweils einer Tablette oder Kapsel dreimal täglich geeignet.

| Bestandteile | Gewicht (mg) Tablette | Kapsel |
|---|---|---|
| 5-(4-Hydroxyphenyl-4-methyl-2-(1H)-pyrimidinon-hydrobromid | 100 | 100 |
| Tragacanth | 10 | – |
| Lactose | 247,5 | 300 |
| Maisstärke | 25 | – |
| Talk | 15 | – |
| Magnesiumstearat | 2,5 | – |

Die in der folgenden Tabelle angegebenen erfindungsgemässen Verbindungen wurden wie angegeben erhalten:

| Beisp. Nr. | $R^1$ | $R^2$ | $R^3$ | Schmp. °C | Salz/Base | hergestellt analog Beispiel Nr. |
|---|---|---|---|---|---|---|
| 4 | $C_2H_5$ | 4-$OCH_3$ | H | 155 | Base | 2 |
| 5 | $C_2H_5$ | 4-OH | H | 302 (Zers.) | Hydrobromid | 1 |
| 6 | $CH_3$ | 2-$OCH_3$ | H | 225 | Hydrochlorid | 2 |
| 7 | $CH_3$ | 3-$OCH_3$ | H | 253 | Hydrochlorid | 2 |
| 8 | $CH_3$ | 3-OH | H | 287 (Zers.) | Hydrobromid | 3 |
| 9 | $CH_3$ | 3-$OCH_3$ | 4-$OCH_3$ | 250 | Hydrochlorid | 2 |
| 10 | $CH_3$ | 3-OH | 4-OH | 289 | Hydrobromid | 1 |

| Beisp. Nr. | $R^1$ | $R^2$ | $R^3$ | Schmp. °C | Salz/Base | hergestellt analog Beispiel Nr. |
|---|---|---|---|---|---|---|
| 11 | $CH_3$ | 2-$OCH_3$ | 5-$OCH_3$ | 170 | Base | 2 |
| 12 | $CH_3$ | 2-OH | 5-OH | 289 | Hydrobromid | 3 |
| 13 | $CH_3$ | 4-$OCH(CH_3)_2$ | H | 256 | Hydrochlorid | 2 |
| 14 | $CH_3$ | H | H | 260 (Zers.) | Hydrochlorid | 2 |
| 15 | $CH_3$ | 2-Cl | 4-Cl | 230 | Base | 2 |
| 16 | $CH_3$ | 4-Cl | H | 203 | Base | 2 |
| 17 | $CH_3$ | 4-$CH_3$ | H | 246 | Base | 2 |
| 18 | $CH_3$ | 4-$CH(CH_3)_2$ | H | 203 | Base | 2 |
| 19 | $CH_3$ | 2-$OCH_3$ | 4-$OCH_3$ | 225 (Zers.) | Hydrochlorid | 2 |
| 20 | $CH_3$ | 4-CN | H | >300 | Base | 2 |
| 21 | $CH_3$ | 3-$CF_3$ | H | 236 | Hydrochlorid | 2 |
| 22 | $CH_3$ | 4-F | H | 255 | Hydrochlorid | 2 |

**Patentansprüche für die Vertragsstaaten BE CH DE FR GB IT LI NL SE**

1. Substituierte Phenyl-2-(1H)-pyrimidinone der allgemeinen Formel I

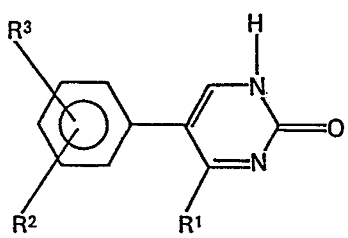

(I)

in der
$R^1$ eine Alkylgruppe mit 1 bis 4 Kohlenstoffatomen und
$R^2$ und $R^3$, die gleich oder verschieden sein können, jeweils Wasserstoff, Halogen, Cyano, eine Trifluormethylgruppe, eine Hydroxylgruppe oder eine Alkoxygruppe mit 1 bis 4 Kohlenstoffatomen bedeuten, wobei Alkylteile jeweils geradkettig oder verzweigt sein können, ihre tautomeren Formen und ihre Säureadditionssalze, sowie ihre N-Oxyde, ausgenommen 5-(2-Hydroxyphenyl)-4-ethyl-2-(1H)-pyrimidinon.

2. 5-(4-Hydroxyphenyl)-4-methyl-2-(1H)-pyrimidinon und dessen Säureadditionssalze.

3. 5-(3-Hydroxyphenyl)-4-methyl-2-(1H)-pyrimidinon und dessen Säureadditionssalze.

4. 5-(4-Methoxyphenyl)-4-methyl-2-(1H)-pyrimidinon und dessen Säureadditionssalze.

5. Verfahren zur Herstellung der Verbindungen der allgemeinen Formel I gemäss Anspruch 1, dadurch gekennzeichnet, dass man Verbindungen der allgemeinen Formel II

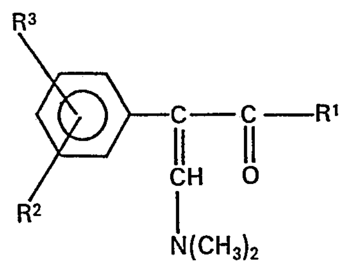

(II)

in der
$R^1$ eine Alkylgruppe mit 1 bis 4 Kohlenstoffatomen und $R^2$ und $R^3$, die gleich der verschieden sein können, jeweils Wasserstoff, Halogen, Cyano, eine Trifluormethylgruppe, eine Hydroxylgruppe oder eine Alkoxygruppe mit 1 bis 4 Kohlenstoffatomen bedeuten, wobei Alkylteile jeweils geradkettig oder verzweigt sein können, mit Harnstoff umsetzt und von den so erhaltenen Verbindungen gegebenenfalls die N-oxyde und die Säureadditionssalze herstellt.

6. Verfahren zur Herstellung der Verbindungen der allgemeinen Formel I gemäss Anspruch 1, in denen $R^2$ und $R^3$ mindestens eine Hydroxylgruppe bedeutet, dadurch gekennzeichnet, dass man die entsprechenden erfindungsgemässen Alkoxyverbindungen einer Etherspaltung unterwirft und von den so erhaltenen Verbindungen gegebenenfalls die N-Oxyde und die Säureadditionssalze herstellt.

7. Substituierte Phenyl-2-(1H)-pyrimidinone der allgemeinen Formel I

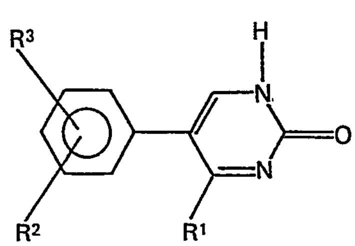

(I)

in der
$R^1$ eine Alkylgruppe mit 1 bis 4 Kohlenstoffatomen und $R^2$ und $R^3$, die gleich oder verschieden sein können, jeweils Wasserstoff, Halogen, Cyano, eine Trifluormethylgruppe, eine Hydroxylgruppe oder eine Alkoxygruppe mit 1 bis 4 Kohlenstoffatomen bedeuten, wobei Alkylteile jeweils geradkettig oder verzweigt sein können, ihre tautomeren Formen und ihre Säureadditionssalze sowie ihre N-Oxyde zur Anwendung als Arzneimittel.

8. Verbindungen der allgemeinen Formel I gemäss Anspruch 7 zur Anwendung bei Herzinsuffizienz.

9. Pharmazeutisches Präparat, dadurch gekennzeichnet, dass es eine oder mehrere der Verbindungen der allgemeinen Formel I gemäss Anspruch 7, deren N-Oxyde oder deren physiologisch verträgliche Säureadditionssalze und gegebenenfalls übliche Trägerstoffe und/oder Verdünnungsmittel enthält.

**Patentansprüche für den Vertragsstaat AT**

1. Verfahren zur Herstellung substituierter Phenyl-2-(1H)-pyrimidinone der allgemeinen Formel I

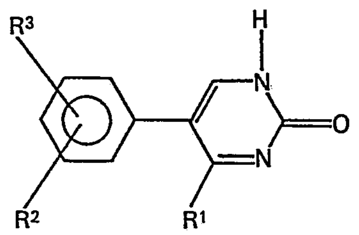

(I)

in der
$R^1$ eine Alkylgruppe mit 1 bis 4 Kohlenstoffatomen und $R^2$ und $R^3$, die gleich oder verschieden sein können, jeweils Wasserstoff, Halogen, Cyano, eine Trifluormethylgruppe, eine Hydroxylgruppe oder eine Alkoxygruppe mit 1 bis 4 Kohlenstoffatomen bedeuten, wobei Alkylteile jeweils geradkettig oder verzweigt sein können, ihrer tautomeren Formen und ihrer Säureadditionssalze sowie ihrer N-Oxyde, dadurch gekennzeichnet, dass man Verbindungen der allgemeinen Formel II

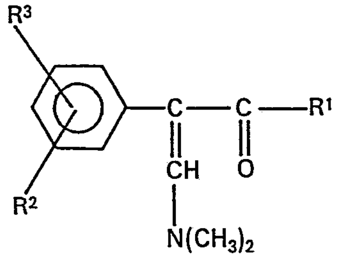

(II)

in der
$R^1$ eine Alkylgruppe mit 1 bis 4 Kohlenstoffatomen und $R^2$ und $R^3$, die gleich der verschieden sein können, jeweils Wasserstoff, Halogen, Cyano, eine Trifluormethylgruppe, eine Hydroxylgruppe oder eine Alkoxygruppe mit 1 bis 4 Kohlenstoffatomen bedeuten, wobei Alkylteile jeweils geradkettig oder verzweigt sein können, mit Harnstoff umsetzt und von den so erhaltenen Verbindungen gewünschtenfalls die N-Oxyde und die Säureadditionssalze herstellt und gewünschtenfalls zur Herstellung der Verbindungen, in denen $R^2$ oder $R^3$ eine Hydroxylgruppe bedeutet, die entsprechenden erfindungsgemässen Alkoxyverbindungen einer Etherspaltung unterwirft und von den so erhaltenen Verbindungen gewünschtenfalls die N-Oxyde oder die Säureadditionssalze herstellt.

2. Verfahren gemäss Anspruch 1 zur Herstellung von 5-(4-Hydroxyphenyl)-4-methyl-2-(1H)-pyrimidinon, dadurch gekennzeichnet, dass man 4-Dimethylamino-3-(4-methoxyphenyl)-3-buten-2-on mit Harnstoff umsetzt und die so erhaltene Methoxyverbindung einer Etherspaltung unterwirft.

3. Verfahren gemäss Anspruch 1 zur Herstellung von 5-(3-Hydroxyphenyl)-4-methyl-2-(1H)-pyrimidinon, dadurch gekennzeichnet, dass man 4-Dimethylamino-3-(3-methoxyphenyl)-3-buten-2-on mit Harnstoff umsetzt und die so erhaltene Methoxyverbindung einer Etherspaltung unterwirft.

4. Verfahren gemäss Anspruch 1 zur Herstellung von 5-(4-Methoxyphenyl)-4-methyl-2-(1H)-pyrimidinon, dadurch gekennzeichnet, dass man 4-Dimethylamino-3-(4-methoxyphenyl)-3-buten-2-on mit Harnstoff umsetzt.

**Claims for the contracting states BE CH DE FR GB IT LI NL SE**

1. Substituted phenyl-2-(1H)-pyrimidinones according to the general formula I

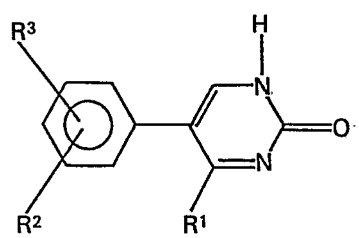

(I)

in which $R^1$ denotes an alkyl group having 1 to 4 carbon atoms and $R^2$ and $R^3$, which can be identical or different, each denote hydrogen, halogen, cyano, a trifluoromethyl group, a hydroxyl group or an alkoxy group having 1 to 4 carbon atoms, it being possible for alkyl moieties in each case to be straight-chain or branched, their tautomeric forms and their acid addition salts, as well as their N-oxids, excepting 5-(2-hydroxyphenyl)-4-ethyl-2-(1H)-pyrimidinone.

2. 5-(4-Hydroxyphenyl)-4-methyl-2-(1H)-pyrimidinone and its acid addition salts.

3. 5-(3-Hydroxyphenyl)-4-methyl-2-(1H)-pyrimidinone and its acid addition salts.

4. 5-(4-Methoxyphenyl)-4-methyl-2-(1H)-pyrimidinone and its acid addition salts.

5. Process for the preparation of compounds of the general formula I according to claim 1, characterized in that compounds of the general formula II

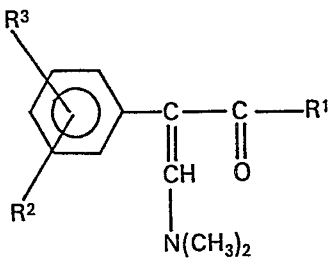

(II)

in which $R^1$ denotes an alkyl group having 1 to 4 carbon atoms, and $R^2$ and $R^3$, which can be identical or different, each denote hydrogen, halogen, cyano, a trifluoromethyl group, a hydroxyl group or an alkoxy group having 1 to 4 carbon atoms, it being possible for alkyl moieties in each case to be straight-chain or branched, are reacted with urea and, where appropriate, the N-oxides and the acid addition salts are prepared from the compounds obtained in this way.

6. Process for the preparation of the compounds of the general formula I according to claim 1, in which $R^2$ and $R^3$ denote at least one hydroxyl group, characterized in that the corresponding alkoxy compounds according to the invention are subjected to an ether cleavage and, where appropriate, the N-oxides and the acid addition salts are prepared from the compounds obtained in this way.

7. Substituted phenyl-2-(1H)-pyrimidinones of the formula I

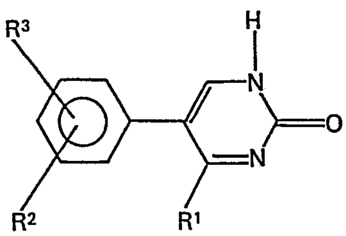

(I)

in which $R^1$ denotes an alkyl group having 1 to 4 carbon atoms and $R^2$ and $R^3$, which can be identical or different, each denote hydrogen, halogen, cyano, a trifluoromethyl group, a hydroxyl group or an alkoxy group having 1 to 4 carbon atoms, it being possible for alkyl moieties in each case to be straight-chain or branched, their tautomeric forms and their acid addition salts, as well as their N-oxides, for use as medicaments.

8. Compounds of the general formula I according to claim 7 for use in cardiac insufficiency.

9. Pharmaceutical product characterized in that it contains one or more of the compounds of the general formula I according to claim 7, their N-oxides or their physiologically tolerated acid addition salts and, where appropriate, customary vehicles and/or diluents.

**Claims for the contracting state AT**

1. Process for the preparation of substituted phenyl-2-(1H)-pyrimidinones of the general formula I

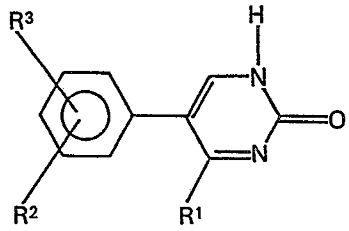

(I)

in which $R^1$ denotes an alkyl group having 1 to 4 carbon atoms and $R^2$ and $R^3$, which can be identical or different, each denote hydrogen, halogen, cyano, a trifluoromethyl group, a hydroxyl group or an alkoxy group having 1 to 4 carbon atoms, it being possible for alkyl moieties in each case to be straight-chain or branched, their tautomeric froms and their acid addition salts, as well as their N-oxides, characterized in that compounds of the general formula II

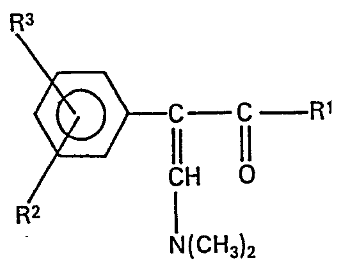

(II)

in which $R^1$ denotes an alkyl group having 1 to 4 carbon atoms, and $R^2$ and $R^3$, which can be identical or different, each denote hydrogen, halogen, cyano, a trifluoromethyl group, a hydroxyl group or an alkoxy group having 1 to 4 carbon atoms, it being possible for alkyl moieties in each case to be straight-chain or branched, are reacted with urea and, if desired, the N-oxides and the acid addition salts are prepared from the compounds obtained in this way, and, if desired for the preparation of the compounds in which $R^2$ or $R^3$ denotes a hydroxyl group, the corresponding alkoxy compounds according to the invention are subjected to an ether cleavage and, if desired, the N-oxids or the acid addition salts are prepared from the compounds prepared in this way.

2. Process according to claim 1 for the preparation of 5-(4-hydroxyphenyl)-4-methyl-2-(1H)-pyrimidinone, characterized in that 4-dimethylamino-3-(4-methoxyphenyl)-3-buten-2-one is reacted with urea, and the methoxy compound obtained in this way is subjected to an ether cleavage.

3. Process according to claim 1 for the preparation of 5-(3-hydroxyphenyl)-4-methyl-2-(1H)-pyrimidinone, characterized in that 4-dimethylamino-3-(3-methoxyphenyl)-3-buten-2-one is reacted with urea, and the methoxy compound obtained in this way is subjected to an ether cleavage.

4. Process according to claim 1 for the preparation of 5-(4-methoxyphenyl)-4-methyl-2-(1H)-pyrimidinone, characterized in that 4-dimethylamino-3-(4-methoxyphenyl)-3-buten-2-one is reacted with urea.

**Revendications pour les états contractants BE CH DE FR GB IT LI NL SE**

1. Phényl-2-(1H)-pyrimidinones substituées de formule générale I

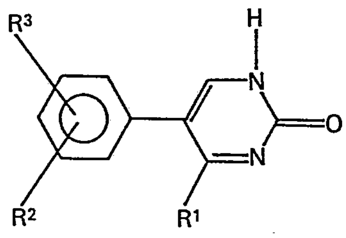

(I)

dans laquelle
$R^1$ désigne un groupe alkyle de 1 à 4 atomes de carbone et $R^2$ et $R^3$ qui peuvent être identiques ou différents, désignent chacun l'hydrogène, un halogène, un groupe cyano, un groupe trifluorométhyle, un groupe hydroxyle, ou un groupe alcoxyle de 1 à 4 atomes de carbone, où les éléments alkyles peuvent être chacun linéaires ou ramifiés, leurs formes tautomères et leurs sels d'addition d'acide, ainsi que leurs N-oxides, à l'exception de la 5-(2-hydroxyphényl)-4-éthyl-2-(1H)-pyrimidinone.

2. 5-(4-Hydroxyphényl)-4-méthyl-2-(1H)-pyrimidinone et ses sels d'addition d'acide.

3. 5-(3-Hydroxyphényl)-4-méthyl-2-(1H)-pyrimidinome et ses sels d'addition d'acide.

4. 5-(4-Méthoxyphényl)-4-méthyl-2-(1H)-pyrimidinone et ses sels d'addition d'acide.

5. Procédé pour la préparation des composés de la formule générale I, selon la revendication 1, caractérisé en ce que l'on fait réagir avec de l'urée des composés de formule générale II

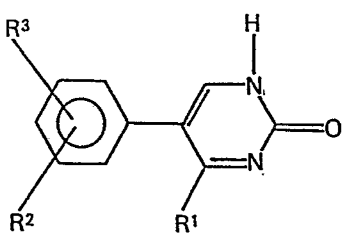

(II)

dans laquelle
$R^1$ désigne un groupe alkyle de 1 à 4 atomes de carbone et $R^2$ et $R^3$, qui peuvent être identiques ou différents, désignent chacun l'hydrogène, un halogène, un groupe cyano, un groupe trifluorométhyle, un groupe hydroxyle ou un groupe alcoxyle de 1 à 4 atomes de carbone, où les éléments alkyles peuvent être linéraires ou ramifiés, et l'on prépare, le cas échéant, à partir des composés ainsi obtenus les N-oxides et les sels d'addition d'acide.

6. Procédé pour la préparation des composés de la formule générale I selon la revendication 1, dans laquelle $R^2$ et $R^3$ désignent au moins un groupe hydroxyle, caractérisé en ce que l'on soumet les composés alcoxylés correspondants selon l'invention à un clivage de l'éther et que l'on prépare le cas échéant à partir des composés ainsi obtenus les N-oxides et les sels d'addition d'acide.

7. Phényl-2-(1H)-pyrimidinones substiuées de formule générale I

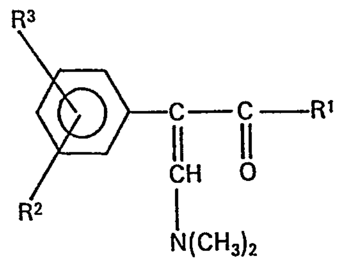

(I)

dans laquelle
$R^1$ désigne un groupe alkyle de 1 à 4 atomes de carbone et $R^2$ et $R^3$ qui peuvent être identiques ou différents, désignent chacun l'hydrogène, un halogène, un groupe cyano, un groupe trifluorométhyle, un groupe hydroxyle ou un groupe alcoxyle de 1 à 4 atomes de carbone, où les éléments alkyles peuvent être chacun linéraires ou ramifiés, leurs formes tautomères et leurs sels d'addition d'acide, ainsi que leurs N-oxides en vue de leur utilisation en tant que médicament.

8. Composés selon la revendication 7 en vue de l'utilisation dans les insuffisances cardiaques.

9. Préparation pharmaceutique caractérisée en ce que'elle contient un où plusieurs des composés de formule générale 1 selon la revendication 7, de leurs N-oxides ou de leurs sels d'addition d'acide physiologiquement perceptables, et le cas échéant, des excépients et/ou des diluants usuels.

**Revendications pour l'état contractant AT**

1. Procédé pour la préparation de phényl-2-(1H)-pyrimidinones substituées de formule générale I

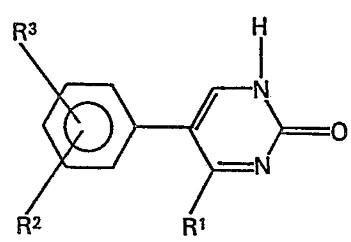

(I)

dans laquelle
$R^1$ désigne un groupe alkyle de 1 à 4 atomes de carbone et $R^2$ et $R^3$ qui peuvent être identiques ou différents, désignent chacun l'hydrogène, un halogène, un groupe cyano, un groupe trifluorométhyle, un groupe hydroxyle ou un groupe alcoxyle de 1 à 4 atomes de carbone, où les éléments alkyles peuvent être chacun linéaires ou ramifiés, leurs formes tautomères et leurs sels d'addition d'acir de ou leurs N-oxides, caractérisé en ce que l'on fait réagir avec de l'urée des composés de formule générale II

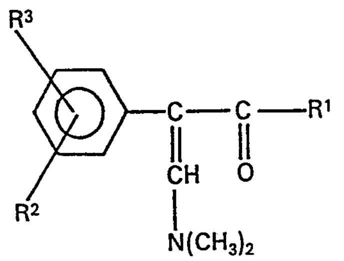

(II)

dans laquelle
$R^1$ désigne un groupe alkyle de 1 à 4 atomes de carbone et $R^2$ et $R^3$ qui peuvent être identiques ou différents, désignent chacun l'hydrogène, un halogène, un groupe cyano, un groupe trifluorométhyle, un groupe hydroxyle ou un groupe alcoxyle de 1 à 4 atomes de carbone, où les éléments alkyles peuvent être chacun linéaires ou ramifiés, et, le cas échéant, on prépare à partir des composés ainsi obtenus les N-oxides et les sels d'addition d'acide et, le cas échéant, en vue de préparer les composés, dans lesquels $R^2$ ou $R^3$ désigne un groupe hydroxyle, l'on soumet les composés alcoxylés correspondants selon l'invention à un clivage de l'éther et que l'on prépare, le cas échéant, à partir des compositions ainsi obtenues les N-oxides ou les sels d'addition d'acide.

2. Procédé selon la revendication 1 pour la préparation de 5-(4-hydroxyphényl)-4-méthyl-2-(1H)-pyrimidinone, caractérisé en ce que l'on fait réagir la 4-diméthylamino-3-(4-méthoxyphényl)-3-butèn-2-one avec de l'urée et que l'on soumet la composition méthoxylée ainsi obtenue à un clivage de l'éther.

3. Procédé selon la revendication 1 pour la préparation de 5-(6-hydroxyphényl)-4-méthyl-2-(1H)-pyrimidinone, caractérisé en ce que l'on fait réagir la 4-diméthylamino-3-(3-méthoxyphényl)-3-butèn-2-one avec de l'urée, et que l'on soumet la composition méthoxylée ainsi obtenue à un clivage de l'éther.

4. Procédé selon la revendication 1 pour la préparation de 5-(4-méthoxyphényl)4-méthyl-2-(1H)-pyrimidinone, caractérisé en ce que l'on fait réagir la 4-diméthylamino-3-(4-méthoxyphényl)-3-butèn-2-one avec de l'urée.